# FASCICULE DE BREVET EUROPEEN

(11) **EP 0 820 990 B1**
(45) Date de publication et mention de la délivrance du brevet: **17.01.2001**
(21) Numéro de dépôt: 97401789.9
(22) Date de dépôt: 24.07.1997
(51) Int. Cl.: C07D 235/12, A61K 31/415, C07D 235/06, C07D 235/10, C07D 235/14, C07D 401/12, C07D 401/14, C07D 401/06, C07D 403/12

(54) **Dérivés de benzimidazole, de benzoxazole et de benzothiazole, utilisables comme inhibiteurs de l'interleukine 1bêta**
Benzimidazol-, Benzoxazol- und Benzothiazolderivate, welche als Inhibitoren des Interleukin 1beta verwendbar sind
Benzimidazole, benzoxazole, and benzothiazole derivatives useful as interleukin 1beta inhibitors

(30) Priorité: 26.07.1996 FR 9609416
(43) Date de publication de la demande: 28.01.1998
(73) Titulaire: ADIR ET COMPAGNIE, 92415 Courbevoie Cédex (FR)
(72) Inventeur: de Nanteuil, Guillaume, 92150 Suresnes (FR); Portevin, Bernard, 78990 Elancourt (FR); Bonnet, Jacqueline, 75013 Paris (FR); Fradin, Armel, 92200 Neuilly Sur Seine (FR)

(56) Documents cités:
- EP-A- 0 260 744
- WO-A-98/05648
- DE-A- 3 313 599
- US-A- 4 430 502
- WOLLWEBER H ET AL: "2-(Guanidino)anilides and related compounds. Synthesis and anthelmintic activity. 3. Anthelmintics" ARZNEIM.-FORSCH. , vol. 34, no. 5, 1984, pages 531-542, XP002028367
- DATABASE WPI Section Ch, Week 9736 Derwent Publications Ltd., London, GB; Class B02, AN 97-389391 XP002067200 & JP 09 169 729 A (GREEN CROSS CORP)

## Description

La présente invention concerne de nouveaux dérivés de benzimidazole, de benzoxazole et de benzothiazole, leur procédé de préparation et les compositions pharmaceutiques qui les contiennent. Ces composés, outre le fait qu'ils soient nouveaux, sont de puissants inhibiteurs de l'interleukine 1β (IL1β).

L'IL1β est produite par les macrophages et possède une grande variété d'activités biologiques reliées à des pathologies inflammatoires comme l'arthrite rhumatoïde ou l'arthrose.
L'IL1β stimule les cellules présentes dans l'articulation qui synthétisent et expriment alors la cyclooxygénase inductible (COX2), ainsi que la NO synthase inductible, pour fournir les prostaglandines et le NO, qui sont des médiateurs importants de la douleur et de l'inflammation. L'IL1β active également l'expression et la synthèse de protéases qui participent à la dégradation de la matrice extracellulaire des chondrocytes et à la suppression de la synthèse des composants de la matrice du cartilage. De plus, l'IL1β intervient dans l'activation des cellules endothéliales qui expriment alors différents facteurs d'adhésion, ainsi que dans l'induction d'autres cytokines pro-inflammatoires comme le TNF ou les chémokines (TIL6). Enfin, l'IL1β joue un rôle dans la régulation de la résorption osseuse ainsi que dans la différentiation et la prolifération lymphocytaires.
On peut donc attendre d'un inhibiteur de l'IL1β qu'il agisse contre les phénomènes inflammatoires et modifie de façon favorable l'évolution de pathologies comme l'arthrite rhumatoïde ou l'arthrose.

Plus spécifiquement, la présente invention concerne les composés de formule (I) : 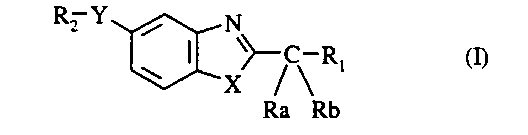 dans laquelle :
- R₁: représente un atome d'halogène, un groupement hydroxy, alkoxy (C₁-C₆) linéaire ou ramifié (éventuellement substitué par un groupement aryle), trihalogénométhyle, amino (éventuellement substitué par un ou plusieurs groupements alkyle (C₁-C₆) linéaire ou ramifié, ou aryle éventuellement substitué), mercapto, alkylthio (C₁-C₆) linéaire ou ramifié, trialkylammonium (C₁-C₆) linéaire ou ramifié, aryloxy, arylthio, arylsulfonyle, arylsulfonyloxy, cycloalkyl (C₃-C₇) oxy, cycloalkyl (C₃-C₇) thio, bicycloalkyle (C₆-C₈) oxy substitué éventuellement par un groupement aryle, bicycloalkyl (C₆-C₈) thio éventuellement substitué par un groupement aryle,
- Rₐ, R_{b},: identiques ou différents, représentent un atome d'hydrogène ou un groupement alkyle (C₁-C₆) linéaire ou ramifié,
- X: représente un atome d'oxygène ou de soufre ou un groupement NR (dans lequel R représente un atome d'hydrogène ou un groupement alkyle (C₁-C₆) linéaire ou ramifié),
- Y: représente un groupement -(CH₂)ₘ-Z-(CH₂)ₙ- dans lequel
m représente 0, 1 ou 2,
n représente 0, 1 ou 2,
Z représente un atome d'oxygène ou de soufre ou un groupement amino (éventuellement substitué par un groupement alkyle (C₁-C₆) linéaire ou ramifié), un groupement -SO₂-, -CHOH- ou -CH(CH₂OH)-,
- R₂: représente un groupement aryle éventuellement substitué,
leurs isomères ainsi que leurs sels d'addition à un acide ou à une base pharmaceutiquement acceptable, à la condition que le composé de formule (I) soit différent du 2-méthoxyméthyl-5-(phénylthio)benzimidazole.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, oxalique, méthane sulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

Par groupement aryle éventuellement substitué, on entend groupement phényle, naphtyle, pyridyle, quinolyle, imidazolyle ou pyridyl-N-oxide, éventuellement substitué par un ou plusieurs atomes d'halogène ou groupements alkyle (C₁-C₆) linéaire ou ramifié, trihalogénoalkyle (C₁-C₆) linéaire ou ramifié, alkoxy (C₁-C₆) linéaire ou ramifié, hydroxy, nitro, cyano, amino (substitué éventuellement par un ou plusieurs groupements alkyle (C₁-C₆) linéaire ou ramifié), phényle substitué ou bicycloalkyle substitué.

Les composés préférés de l'invention sont les composés de formule (I) dans lesquels X représente un groupement -NR (dans lequel R représente un atome d'hydrogène ou un groupement alkyle (C₁-C₆) linéaire ou ramifié).

Les groupements R₂ préférés selon l'invention sont les groupements phényle et pyridyle, chacun de ces groupements étant éventuellement substitués.

Les groupements R₁ préférés selon l'invention sont les groupements hydroxy, mercapto, aryloxy et arylthio.

Les composés préférés selon l'invention sont les composés de formule (I) dans lesquels Y représente un atome d'oxygène, de soufre ou un groupement amino éventuellement substitué.

La présente invention s'étend également au procédé de préparation des composés de formule (I). Lorsque les composés de formule (I) que l'on souhaite obtenir sont tels que X = NR, le procédé est caractérisé en ce que l'on utilise comme produit de départ un composé de formule (II) :

R₂ - Y - H (II)

dans laquelle R₂ et Y sont tels que définis dans la formule( I), que l'on fait réagir sur la 2-nitro-5-chloroaniline,
pour conduire au composé de formule (III) : 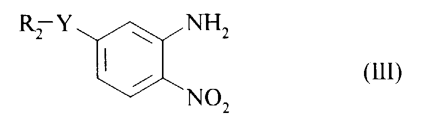 dans laquelle R₂ et Y sont tels que définis dans la formule (I), qui subit une réduction catalytique,
pour conduire au composé de formule (IV) : 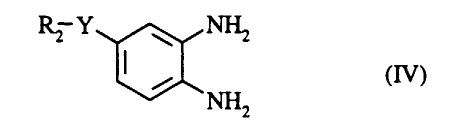 dans laquelle R₂ et Y sont tels que définis dans la formule (I),
que l'on fait réagir, en milieu acide, avec un composé de formule (V) : 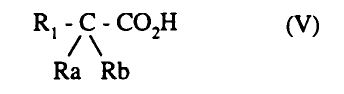 dans laquelle Rₐ, R_{b} et R₁ ont la même signification que dans la formule (I),
pour conduire au composé de formule (I/a), cas particulier des composés de formule (I) : 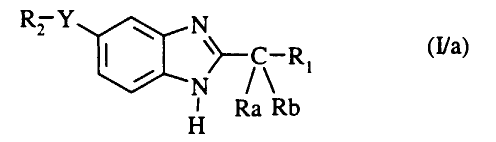 dans laquelle R₁, Rₐ, R_{b}, R₂ et Y sont tels que définis dans la formule (I),
qui subit, éventuellement lorsque R₁ représente un groupement hydroxy, l'action du chlorure de thionyle,
pour conduire au composé de formule (I/b), cas particulier des composés de formule (I) : 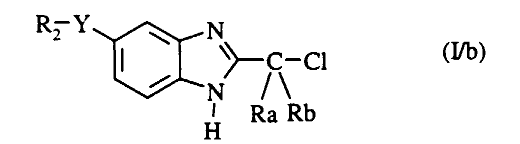 dans laquelle R₂, Y, Rₐ et R_{b} ont la même signification que dans la formule (I),
qui peut alors subir les réactions classiques que l'on peut mettre en oeuvre sur des dérivés chlorés, pour obtenir les substitutions correspondantes,
composé de formule (I/a) ou (I/b) :
- dont la fonction NH peut être éventuellement substituée par un groupement alkyle (C₁-C₆) linéaire ou ramifié,
- qui peut être, le cas échéant, purifié selon une technique classique de purification
- dont on sépare, le cas échéant, les isomères selon une technique classique de séparation,
- que l'on transforme, si on le souhaite, en ses sels d'addition à une base pharmaceutiquement acceptable.

Les composés de formule (III) décrits précédemment peuvent également être obtenus en faisant réagir un dérivé halogéné de formule R₂-Y-hal (tels que R₂ et Y sont tels que définis dans la formule (I) et hal représente un atome d'halogène) sur une hydroxynitroaniline.

Lorsque les composés de formule (I) que l'on souhaite obtenir sont tels que X= X' = O ou S, le procédé est caractérisé en ce que l'on utilise comme produit de départ un composé de formule (VI): 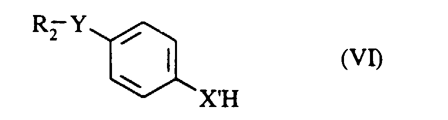 dans lequel R₂ et Y sont tels que définis dans la formule (I) et X' représente un atome d'oxygène ou de soufre, sur lequel on fait réagir de l'acide nitrique,
pour conduire au composé de formule (VII) : 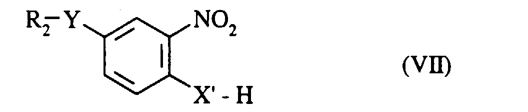 dans laquelle R₂, Y et X' sont tels que définis précédemment, qui subit une hydrogénation catalytique,
pour conduire au composé de formule (VIII): 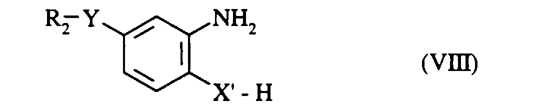 que l'on fait réagir, en milieu acide, avec un composé de formule (V) : 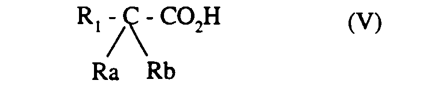 dans laquelle Rₐ, R_{b} et R₁ ont la même signification que dans la formule (I),
pour conduire au composé de formule (I/c), cas particulier des composés de formule (I) : 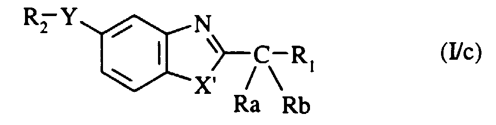 dans laquelle R₂, Y, X', Rₐ, R_{b} et R₁ sont tels que définis précédemment,
qui subit, éventuellement lorsque R₁ représente un groupement hydroxy, l'action du chlorure de thionyle,
pour conduire au composé de formule (I/d), cas particulier des composés de formule (I) : 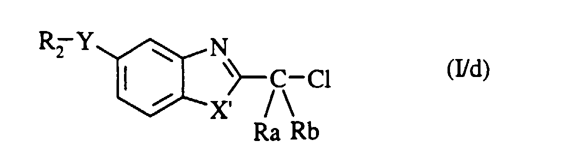 dans laquelle R₂, X', Y, Rₐ et R_{b} ont la même signification que précédemment,
qui peut alors subir les réactions classiques que l'on peut mettre en oeuvre sur des dérivés chlorés, pour obtenir les substitutions correspondantes,
composé de formule (I/c) ou (I/d) :
- qui peut être, le cas échéant, purifié selon une technique classique de purification
- dont on sépare, le cas échéant, les isomères selon une technique classique de séparation,
- que l'on transforme, si on le souhaite, en ses sels d'addition à une base pharmaceutiquement acceptable.

L'invention s'étend aussi aux compositions pharmaceutiques renfermant comme principe actif au moins un composé de formule (I) avec un ou plusieurs excipients inertes, non toxiques et appropriés. Parmi les compositions pharmaceutiques selon l'invention, on pourra citer plus particulièrement celles qui conviennent pour l'administration orale, parentérale (intraveineuse ou sous-cutanée), nasale, les comprimés simples ou dragéifiés, les comprimés sublinguaux, les gélules, les tablettes, les suppositoires, les crèmes, les pommades, les gels dermiques, les préparations injectables, les suspensions buvables, etc...

La posologie utile est adaptable selon la nature et la sévérité de l'affection, la voie d'administration ainsi que selon l'âge et le poids du patient. Cette posologie varie de 0,1 à 100 mg par jour en une ou plusieurs prises.

Les exemples suivants illustrent l'invention mais ne la limitent en aucune façon.

Les produits de départ utilisés sont des produits connus ou préparés selon des modes opératoires connus.

Les structures des composés décrits dans les exemples et les préparations ont été déterminées selon les techniques spectrophotométriques usuelles (infrarouge, RMN, spectrométrie de masse,...).

### EXEMPLE 1 : 2-Hydroxyméthyl-5-(4-pyridyloxy)benzimidazole

### Stade 1 : 2-Amino-4-(4-pyridyloxy)nitrobenzène

Dans un ballon de 2 litres, on charge 35,2 g (0,369 moles) de 4-hydroxypyridine et 250 ml de diméthylformamide (DMF) anhydre. Sous azote, on ajoute par fractions 42 g (0,369 moles) de terbutylate de potassium en maintenant la température à 15-20°C par un bain eau-glace. Après addition, on agite 2 heures, on obtient une solution jaune pale. On ajoute ensuite 60 g (0,358 moles) de 2-nitro-5-chloroaniline, on obtient une coloration rouge intense. On porte 6 heures à 100°C puis on laisse refroidir la nuit. On évapore le DMF puis on ajoute de l'eau ; on obtient un précipité qui est filtré et lavé par de l'isopropanol à chaud puis séché.
*Point de fusion :* > *260°C*

| *Microanalyse élémentaire :* | | | |
|---|---|---|---|
| | *C %* | *H %* | *N %* |
| *calculé* | *57,14* | *3,92* | *18,17* |
| *trouvé* | *56,93* | *3,98* | *17,75* |

### Stade 2 : 2-Amino-4-(4-pyridyloxy)aniline

30 g (0,130 moles) de 2-amino-4-(4-pyridyloxy)nitrobenzène obtenus au stade précédent sont mis en suspension dans 600 ml d'un mélange eau/acide citrique (50/50). On ajoute 2 g de palladium sur charbon 10 % et on hydrogène 18 h sous 4 kg à température ambiante. On filtre le catalyseur et on évapore le filtrat. Le résidu est dissous dans l'eau. Cette phase aqueuse est alcalinisée par du carbonate de potassium. Le précipité obtenu est filtré, lavé à l'eau et séché. Le produit attendu est purifié par chromatographie sur gel de silice (éluant : CH₂Cl₂ / MeOH : 80/20).
*Point de fusion : 260°C*

| *Microanalyse élémentaire :* | | | |
|---|---|---|---|
| | *C %* | *H %* | *N %* |
| *calculé* | *65,66* | *5,51* | *20,88* |
| *trouvé* | *65,78* | *5,54* | *20,79* |

### Stade 3 : 2-Hydroxyméthyl-5-(4-pyridyloxy)benzimidazole

Dans un ballon de 250 ml muni d'une agitation et d'un réfrigérant, on charge 3 g (14,9 mmoles) du composé obtenu au stade précédent, 30 ml d'acide chlorhydrique 4N et 2,1 g (50 % excès) d'acide glycolique. L'ensemble est porté 6 heures à reflux puis laissé refroidi. Par alcalinisation par de la soude 10N, le produit précipite et est filtré, lavé par 3 fois 30 ml d'eau et séché au dessicateur. Le produit est purifié par chromatographie sur gel de silice (éluant CHCl₂/MeOH/NH₂OH : 80/20/1).
*Point de fusion : 250°C*

| *Microanalyse élémentaire :* | | | |
|---|---|---|---|
| | *C %* | *H %* | *N %* |
| *calculé* | *64,72* | *4,60* | *17,42* |
| *trouvé* | *64,60* | *5,04* | *17,35* |

### EXEMPLE 2 : 2-Chlorométhyl-5-(4-pyridyloxy)benzimidazole, dichlorhydrate

Dans un ballon de 250 ml muni d'une agitation et d'un réfrigérant, on charge 3,18 g (13,2 mmoles) de 2-hydroxyméthyl-5-(4-pyridyloxy)benzimidazole obtenu dans l'exemple 1 et 40 ml de chlorure de thionyle. L'ensemble est porté à reflux 2 heures puis refroidi. Le précipité est filtré, lavé à l'éther, séché et conduit au produit attendu.
*Point de fusion : > 250°C*

| *Microanalyse élémentaire :* | | | | | |
|---|---|---|---|---|---|
| | *C %* | *H %* | *N %* | *Cl %* | *Cl*^{*-*} *%* |
| *calculé* | *46,94* | *3,64* | *12,63* | *31,98* | *21,32* |
| *trouvé* | *46,13* | *3,62* | *12,09* | *32,04* | *21,06* |

### EXEMPLE 3 : 2-Hydroxyméthyl-5-phénoxybenzimidazole

### Stade 1 : 2-Amino-4-phénoxynitrobenzène

Le produit attendu est obtenu selon le procédé décrit au stade 1 de l'exemple 1 en remplaçant la 4-hydroxypyridine par le phénol.
*Point de fusion : 150°C*

### Stade 2 : 2-Amino-4-phénoxyaniline

23,6 g (0,109 moles) du 2-amino-4-phénoxynitrobenzène obtenu au stade précédent sont dissous dans 550 ml de dioxane. Après hydrogènation (H₂/Pd) 18 h à température ambiante sous 4 kg, on filtre le catalyseur et on évapore le filtrat. Le résidu est repris par du pentane, filtré et séché et conduit au produit attendu.
*Point de fusion : 72 °C*

### Stade 3 : 2-Hydroxyméthyl-5-phénoxybenzimidazole

19,4 g (97 mmoles) du composé obtenu au stade précédent, 155 ml d'HCl 4N et 14,2 g (50 % excès) d'acide glycolique sont porté 5 heures à reflux sous agitation. On laisse refroidir. Le chlorhydrate du produit attendu précipite, est filtré, rincé à l'eau et séché.
*Point de fusion : 220°C*

| *Microanalyse élémentaire :* | | | | | |
|---|---|---|---|---|---|
| | *C %* | *H %* | *N %* | *Cl %* | *Cl*^{*-*} *%* |
| *calculé* | *60,77* | *4,73* | *10,12* | *12,81* | *12,81* |
| *trouvé* | *60,67* | *4,76* | *10,07* | *12,81* | *12,98* |

Par alcalinisation du chlorhydrate par de la soude 10N, on récupère après filtration, lavage à l'eau et séchage, le produit attendu sous forme de base libre.
*Point de fusion : 200°C*

### EXEMPLE 4 : Chlorhydrate de 2-chlorométhyl-5-phénoxybenzimidazole

3,6 g (15 mmoles) du composé décrit dans l'exemple 3, 60 ml de toluène et 8 ml de chlorure de thionyle sont portés 1 h 30 au reflux. Après filtration et séchage, on obtient le produit attendu.
*Point de fusion : 172°C*

| *Microanalyse élémentaire :* | | | | | |
|---|---|---|---|---|---|
| | *C %* | *H %* | *N %* | *Cl %* | *Cl*^{*-*} *%* |
| *calculé* | *56,97* | *4,10* | *9,49* | *24,02* | *12,01* |
| *trouvé* | *56,06* | *4,08* | *9,30* | *23,86* | *11,92* |

### EXEMPLE 5 : Chlorure de (5-phénoxybenzimidazol-2-yl)méthyltriméthylammonium

2 g (6,8 mmoles) du composé décrit dans l'exemple 4 sont mis en suspension dans 100 ml d'acétone. On ajoute 6,6 g d'une solution 33 % de triméthylamine dans l'éthanol. Le milieu se solubilise instantanément. Après une nuit d'agitation à température ambiante, l'ensemble est évaporé. Le résidu est purifié sur colonne Biogel (CH₃CN / H₂O : 50/50) puis cristallisé dans l'acétone. Le produit attendu est obtenu après filtration et séchage.
*Point de fusion : 238-240°C*

| *Microanalyse élémentaire :* | | | | |
|---|---|---|---|---|
| | *C %* | *H %* | *N %* | *Cl*^{*-*} *%* |
| *calculé* | *64,25* | *6,34* | *13,22* | *11,16* |
| *trouvé* | *64,19* | *6,49* | *12,86* | *11,02* |

Les exemples suivants ont été obtenus selon le procédé décrit dans l'exemple 3 à partir des produits de départ correspondants.

### EXEMPLE 6 : 2-Mercaptométhyl-5-phénoxybenzimidazole

A partir d'acide mercaptoéthanoïque.
*Point de fusion : 168°C*

| *Microanalyse élémentaire :* | | | | | |
|---|---|---|---|---|---|
| | *C %* | *H %* | *N %* | *Cl %* | *S %* |
| *calculé* | *57,43* | *4,48* | *9,57* | *12,11* | *10,95* |
| *trouvé* | *57,27* | *4,53* | *9,27* | *12,40* | *10,49* |

### EXEMPLE 7 : 2-Aminométhyl-5-phénoxybenzimidazole

A partir de la glycine.
*Point de fusion : 123°C*

| *Microanalyse élémentaire :* | | | |
|---|---|---|---|
| | *C %* | *H %* | *N %* |
| *calculé* | *70,28* | *5,48* | *17,56* |
| *trouvé* | *69,96* | *5,67* | *17,26* |

### EXEMPLE 8 : 2-(Trifluorométhyl)méthyl-5-phénoxybenzimidazole

A partir d'acide trifluorométhyléthanoïque.
*Point de fusion : 188°C*

### EXEMPLE 9 : 2-(Méthoxy)méthyl-5-phénoxybenzimidazole

A partir d'acide méthoxyéthanoïque.
*Point de fusion : 74°C*

| *Microanalyse élémentaire :* | | | |
|---|---|---|---|
| | *C %* | *H %* | *N %* |
| *calculé* | *70,85* | *5,55* | *11,02* |
| *trouvé* | *70,93* | *5,85* | *10,89* |

### EXEMPLE 10 : 2-Tosyloxyméthyl-5-phénoxybenzimidazole

Par réaction du composé décrit dans l'exemple 3 avec le chlorure de tosyle.
*Point de fusion : 138°C*

| *Microanalyse élémentaire :* | | | | |
|---|---|---|---|---|
| | *C %* | *H %* | *N %* | *S %* |
| *calculé* | *63,95* | *4,60* | *7,10* | *8,13* |
| *trouvé* | *64,02* | *4,67* | *7,09* | *7,98* |

### EXEMPLE 11 : 2-(2-Hydroxypropyl-2)-5-phénoxybenzimidazole

*Point de fusion : 186°C*

| *Microanalyse élémentaire :* | | | |
|---|---|---|---|
| | *C %* | *H %* | *N %* |
| *calculé* | *71,62* | *6,01* | *10,44* |
| *trouvé* | *71,96* | *6,11* | *10,28* |

### EXEMPLE 12 : Dichlorhydrate de 2-hydroxyméthyl-5-(4-anilyloxy)benzimidazole

*Point de fusion : > 250°C*

| *Microanalyse élémentaire :* | | | | |
|---|---|---|---|---|
| | *C %* | *H %* | *N %* | *Cl %* |
| *calculé* | *51,24* | *4,61* | *12,80* | *21,60* |
| *trouvé* | *51,02* | *4,99* | *12,26* | *21,01* |

### EXEMPLE 13 : Dichlorhydrate de 5-(1-phényl-1-aminométhyl)-2-hydroxyméthyl benzimidazole

*Point de fusion : 200°C*

| *Microanalyse élémentaire :* | | | | |
|---|---|---|---|---|
| | *C %* | *H %* | *N %* | *Cl %* |
| *calculé* | *55,23* | *5,25* | *12,88* | *21,74* |
| *trouvé* | *55,82* | *5,39* | *12,74* | *22,58* |

### EXEMPLE 14 : Chlorhydrate de 5-(1-phényl-1-hydroxyméthyl)-2-hydroxyméthyl benzimidazole

*Point de fusion : > 260°C*

| *Microanalyse élémentaire :* | | | | |
|---|---|---|---|---|
| | *C %* | *H %* | *N %* | *Cl %* |
| *calculé* | *61,97* | *5,20* | *9,63* | *12,19* |
| *trouvé* | *62,04* | *5,27* | *9,52* | *12,66* |

### EXEMPLE 15 : Dichlorhydrate de 2-hydroxyméthyl-5-(4-pyridylthio)benzimidazole

*Point de fusion : > 260°C*

| *Microanalyse élémentaire :* | | | | | |
|---|---|---|---|---|---|
| | *C %* | *H %* | *N %* | *Cl %* | *S %* |
| *calculé* | *47,28* | *3,97* | *12,72* | *21,47* | *9,71* |
| *trouvé* | *47,16* | *3,92* | *12,24* | *21,65* | *9,93* |

### EXEMPLE 16 : Dichlorhydrate de 2-hydroxyméthyl-5-(3-pyridyloxy)benzimidazole

*Point de fusion : 185°C*

| *Microanalyse élémentaire :* | | | | |
|---|---|---|---|---|
| | *C %* | *H %* | *N %* | *Cl %* |
| *calculé* | *49,70* | *4,17* | *13,37* | *22,57* |
| *trouvé* | *49,30* | *4,97* | *13,05* | *22,59* |

### EXEMPLE 17 : Dichlorhydrate de 2-hydroxyméthyl-5-(2-pyridylthio)benzimidazole

*Point de fusion : 190°C*

| *Microanalyse élémentaire :* | | | | | |
|---|---|---|---|---|---|
| | *C %* | *H %* | *N %* | *Cl %* | *S %* |
| *calculé* | *47,28* | *3,97* | *12,72* | *21,47* | *9,71* |
| *trouvé* | *46,95* | *4,05* | *12,63* | *21,67* | *9,96* |

### EXEMPLE 18 : Dichlorhydrate de 2-phénoxyméthyl 5-(4-pyridylthio)benzimidazole

*Point de fusion : 160°C*

| *Microanalyse élémentaire :* | | | | | |
|---|---|---|---|---|---|
| | *C %* | *H %* | *N %* | *Cl %* | *S %* |
| *calculé* | *56,16* | *4,22* | *10,34* | *17,45* | *7,89* |
| *trouvé* | *57,27* | *4,09* | *10,31* | *17,58* | *7,89* |

### EXEMPLE 19 : 2-Hydroxyméthyl-5-[(7-trifluorométhylquinoléin-4-yl)thio] benzimidazole

*Point de fusion : 242°C*

| *Microanalyse élémentaire :* | | | | |
|---|---|---|---|---|
| | *C %* | *H %* | *N %* | *S %* |
| *calculé* | *57,60* | *3,22* | *11,19* | *8,54* |
| *trouvé* | *57,68* | *3,54* | *10,80* | *8,21* |

### EXEMPLE 20 : Dichlorhydrate de 2-hydroxyméthyl-5-[(imidazol-2-yl)thio] benzimidazole

*Point de fusion : > 260°C*

| *Microanalyse élémentaire :* | | | | | |
|---|---|---|---|---|---|
| | *C %* | *H %* | *N %* | *Cl %* | *S %* |
| *calculé* | *41,39* | *3,79* | *17,55* | *22,21* | *10,04* |
| *trouvé* | *41,36* | *3,88* | *17,08* | *22,45* | *10,05* |

### EXEMPLE 21 : Trichlorhydrate de 2-(4-pyridylthiométhyl)-5-(4-pyridylthio) benzimidazole

*Point de fusion :* > *260°C*

| *Microanalyse élémentaire :* | | | | | |
|---|---|---|---|---|---|
| | *C %* | *H %* | *N %* | *Cl %* | *S %* |
| *calculé* | *47,01* | *3,73* | *12,19* | *23,13* | *13,93* |
| *trouvé* | *47,31* | *4,01* | *12,16* | *23,27* | *14,10* |

### EXEMPLE 22 : Dichlorhydrate de 2-phénylthiométhyl-5-(4-pyridylthio)benzimidazole

*Point de fusion : 168°C*

| *Microanalyse élémentaire :* | | | | | |
|---|---|---|---|---|---|
| | *C %* | *H %* | *N %* | *Cl %* | *S %* |
| *calculé* | *54,03* | *4,06* | *9,95* | *16,79* | *15,18* |
| *trouvé* | *53,61* | *4,24* | *9,71* | *17,28* | *14,82* |

### EXEMPLE 23 : Dichlorhydrate 2-hydroxyméthyl-5-(3-pyridylthio)benzimidazole

*Point de fusion : 260°C*

| *Microanalyse élémentaire :* | | | | | |
|---|---|---|---|---|---|
| | *C %* | *H %* | *N %* | *Cl %* | *S %* |
| *calculé* | *47,28* | *3,97* | *12,72* | *21,47* | *9,71* |
| *trouvé* | *46,41* | *3,86* | *12,34* | *22,57* | *9,58* |

### EXEMPLE 24 : Dichlorhydrate de 2-(4-chlorophénoxyméthyl)-5-(4-pyridylthio) benzimidazole

*Point de fusion : 198°C*

| *Microanalyse élémentaire :* | | | | | |
|---|---|---|---|---|---|
| | *C %* | *H %* | *N %* | *Cl %* | *S %* |
| *calculé* | *51,77* | *3,66* | *9,53* | *24,13* | *7,27* |
| *trouvé* | *52,43* | *3,71* | *9,60* | *24,24* | *6,93* |

### EXEMPLE 25 : Dichlorhydrate de 2-(phénylsulfonylméthyl)-5-(4-pyridylthio) benzimidazole

| *Microanalyse élémentaire :* | | | | | |
|---|---|---|---|---|---|
| | *C %* | *H %* | *N %* | *Cl %* | *S %* |
| *calculé* | *50,22* | *3,77* | *9,25* | *15,60* | *14,11* |
| *trouvé* | *50,54* | *3,97* | *9,22* | *16,09* | *14,17* |

### EXEMPLE 26 : 2-Phénoxyméthyl-5-(4-pyridylsulfonyl-N-oxide)benzimidazole

*Point de fusion : 210°C*

| *Microanalyse élémentaire :* | | | | |
|---|---|---|---|---|
| | *C %* | *H %* | *N %* | *S %* |
| *calculé* | *59,83* | *3,96* | *11,02* | *8,41* |
| *trouvé* | *59,68* | *3,99* | *11,04* | *8,13* |

### EXEMPLE 27 : Dichlorhydrate de 2-phénoxyméthyl-5-(4-pyridylsulfonyl)benzimidazole

*Point de fusion : 174°C*

| *Microanalyse élémentaire :* | | | | | |
|---|---|---|---|---|---|
| | *C %* | *H %* | *N %* | *Cl %* | *S %* |
| *calculé* | *52,06* | *3,92* | *9,59* | *16,18* | *7,31* |
| *trouvé* | *52,27* | *4,01* | *9,30* | *15,44* | *6,64* |

### EXEMPLE 28 : 2-Benzyloxyméthyl-5-(4-pyridylthio)benzimidazole

| *Microanalyse élémentaire :* | | | | |
|---|---|---|---|---|
| | C % | H % | N % | S % |
| *calculé* | *69,14* | *4,93* | *12,09* | *9,23* |
| *trouvé* | *69,43* | *4,92* | *12,08* | *9,62* |

### EXEMPLE 29 : 2-(2-Naphtyloxyméthyl)-5-(4-pyridylthio)benzimidazole

| *Microanalyse élémentaire :* | | | | |
|---|---|---|---|---|
| | C% | *H%* | N% | S% |
| *calculé* | *72,04* | *4,47* | *10,96* | *8,30* |
| *trouvé* | *72,26* | *4,41* | *11,00* | *8,30* |

### EXEMPLE 30 : 2-(1-Naphtyloxyméthyl)-5-(4-pyridylthio)benzimidazole

| *Microanalyse élémentaire :* | | | | |
|---|---|---|---|---|
| | C% | H% | N% | S% |
| *calculé* | *72,04* | *4,47* | *10,96* | *8,36* |
| *trouvé* | *72,46* | *4,41* | *10,80* | *8,57* |

### EXEMPLE 31 : Dichlorhydrate de 2-phénoxyméthyl-5-(N-méthyl 4-pyridylamino) benzimidazole

| *Microanalyse élémentaire :* | | | | |
|---|---|---|---|---|
| | *C %* | *H %* | *N %* | *Cl %* |
| *calculé* | *59,56* | *5,00* | *13,89* | *17,58* |
| *trouvé* | *59,32* | *5,01* | *13,79* | *18,61* |

### EXEMPLE 32 : 2-Phénoxyméthyl-5-(3-pyridylthio)benzimidazole

*Point de fusion : 118°C*

| *Microanalyse élémentaire :* | | | | |
|---|---|---|---|---|
| | C% | *H%* | *N%* | S% |
| *calculé* | *68,45* | *4,53* | *12,60* | *9,62* |
| *trouvé* | *68, 77* | *4,60* | 12,68 | 9,82 |

### EXEMPLE 33 : 2-Phénoxyméthyl-5-((R,S)-α-hydroxybenzyl)benzimidazole

| *Microanalyse élémentaire :* | | | |
|---|---|---|---|
| | *C%* | *H %* | *N%* |
| calculé | 76,34 | 5,49 | 8,48 |
| trouvé | 76,04 | 5,54 | 8,29 |

### EXEMPLE 34 : Dichlorhydrate de 2-hydroxyméthyl-5-(4-pyridylamino)benzimidazole

*Point de fusion : > 250°C*

| *Microanalyse élémentaire :* | | | | |
|---|---|---|---|---|
| | *C %* | *H %* | *N %* | *Cl %* |
| *calculé* | *49,86* | *4,54* | *17,89* | *22,64* |
| *trouvé* | *49,89* | *4,56* | *17,87* | *22,42* |

Les exemples 35 à 37 ont été synthétisés selon le procédé décrit dans l'exemple 3 en faisant réagir, au stade 1, la 5-hydroxy-2-nitroaniline sur le dérivé halogéné correspondant.

### EXEMPLE 35 : Dichlorhydrate de 2-hydroxyméthyl-5-(4-pyridyloxy)benzimidazole

*Point de fusion : 220°C*

| *Microanalyse élémentaire :* | | | | |
|---|---|---|---|---|
| | *C %* | *H %* | *N %* | *Cl %* |
| *calculé* | *49,70* | *4,17* | *13,37* | *22,57* |
| *trouvé* | *49,51* | *4,31* | *13,22* | *22,81* |

### EXEMPLE 36 : 2-Phénoxyméthyl-5-(4-pyridylméthoxy)benzimidazole

*Point de fusion : 162°C*

| *Microanalyse élémentaire :* | | | |
|---|---|---|---|
| | *C %* | *H %* | *N %* |
| *calculé* | *72,49* | *5,17* | *12,68* |
| *trouvé* | *72,34* | *5,32* | *12,69* |

### EXEMPLE 37 : Dichlorhydrate de 2-hydroxyméthyl-5-(4-pyridylméthoxy) benzimidazole

*Point de fusion : > 260°C*

| *Microanalyse élémentaire :* | | | | |
|---|---|---|---|---|
| | *C %* | *H %* | *N %* | *Cl %* |
| *calculé* | *51,24* | *4,61* | *12,80* | *21,60* |
| *trouvé* | *51,19* | *5,09* | *12,88* | *21,49* |

### EXEMPLE 38 : Dichlorhydrate de 2-phénoxyméthyl-5-(4-pyridylméthyl)benzimidazole

Le composé attendu a été synthétisé selon le procédé décrit dans l'exemple 3, stades 2 et 3, en utilisant au stade 2 la 2-nitro-4-(4-pyridylméthyl)aniline.
*Point de fusion : 160°C*

| *Microanalyse élémentaire :* | | | | |
|---|---|---|---|---|
| | *C %* | *H %* | *N %* | *Cl %* |
| *calculé* | *61,87* | *4,93* | *10,82* | *18,26* |
| *trouvé* | *62,03* | *5,08* | *10,87* | *18,62* |

Les exemples 39 et 40 ont été obtenus par méthylation du composé de l'exemple 3 puis séparation par chromatographie sur colonne de silice.

### EXEMPLE 39 : Chlorhydrate de 1-méthyl-2-hydroxyméthyl-5-phénoxy benzimidazole

*Point de fusion : 194°C*

| *Microanalyse élémentaire :* | | | | |
|---|---|---|---|---|
| | *C %* | *H %* | *N %* | *Cl %* |
| *calculé* | *61,97* | *5,20* | *9,63* | *12,19* |
| *trouvé* | *61,88* | *5,22* | *9,54* | *12,01* |

### EXEMPLE 40 : Chlorhydrate de 3-méthyl-2-hydroxyméthyl-5-phénoxy benzimidazole

*Point de fusion : 164°C*

| *Microanalyse élémentaire :* | | | | |
|---|---|---|---|---|
| | *C %* | *H %* | *N %* | *Cl %* |
| *calculé* | *61,97* | *5,20* | *9,63* | *12,19* |
| *trouvé* | *62,05* | *5,06* | *9,60* | *12,28* |

Les composés des exemples suivants ont été préparés à partir des produits de départ correspondants.

### EXEMPLE 41 : 2-Phénoxyméthyl-5-(4-pyridylthiométhyl)benzimidazole

### EXEMPLE 42 : 2-Cyclohexyloxyméthyl-5-(4-pyridylthio)benzimidazole

### EXEMPLE 43 : 2-(4-Chlorophénoxy)méthyl-5-(4-pyridylthio)benzimidazole

### EXEMPLE 44 : 2-(3,4-Dichlorophénoxy)méthyl-5-(4-pyridylthio)benzimidazole

### EXEMPLE 45 : 2-(2,4-Dichlorophénoxy)méthyl-5-(4-pyridylthio)benzimidazole

### EXEMPLE 46 : 2-(4-Méthoxyphénoxy)méthyl-5-(4-pyridylthio)benzimidazole

### EXEMPLE 47 : 2-(4-Fluorophénoxy)méthyl-5-(4-pyridylthio)benzimidazole

### EXEMPLE 48 : 2-(3,4,5-Triméthoxyphénoxy)méthyl-5-(4-pyridylthio)benzimidazole

### EXEMPLE 49 : 2-(2,6-Diméthoxyphénoxy)méthyl-5-(4-pyridylthio)benzimidazole

### EXEMPLE 50 : 2-[3-(Trifluorométhyl)phénoxy]méthyl-5-(4-pyridylthio)benzimidazole

### EXEMPLE 51 : 2-[3,5-Bis(trifluorométhyl)phénoxy]méthyl-5-(4-pyridylthio) benzimidazole

### EXEMPLE 52 : 2-[(4-Phényl)phénoxy]méthyl-5-(4-pyridylthio)benzimidazole

### EXEMPLE 53 : 2-[4-(4-Méthylphényl)bicyclo[2.2.2]oct-1-yloxy]méthyl-5-(4-pyridylthio)benzimidmole

### EXEMPLE 54 : 2-[4-(4-Méthoxybicyclo[2.2.2]oct-1-yl)phénoxy]-méthyl-5-(4-pyridylthio)benzimidazole

### EXEMPLE 55 : 2-Phénylthiométhyl-5-(3-pyridylthio)benzimidazole

### EXEMPLE 56 : 2-Phénylsulfonylméthyl-5-(4-pyridylthio)benzimidazole

### EXEMPLE 57 : 2-(4-Pyridyloxy)méthyl-5-(4-pyridylthio)benzimidazole

### EXEMPLE 58 : 2-Phénoxyméthyl-5-(1-phényl-1-hydroxyméthyl)benzimidazole

### EXEMPLE 59 : 2-(4-Pyridyloxy)méthyl-5-(1-phényl-1-hydroxyméthyl)benzimidazole

### EXEMPLE 60 : 2-Phénoxyméthyl-5-[1-(3-pyridyl)-2-hydroxyéthyl]benzimidazole

### EXEMPLE 61 : 2-(4-Pyridyloxy)méthyl-5-[1-(3-pyridyl)-2-hydroxyéthyl]benzimidazole

### EXEMPLE 62 : 2-Phénoxyméthyl-5-[(3-pyridyl)méthylthiométhyl]benzimidazole

### EXEMPLE 63 : 2-Phénoxyméthyl-5-[2-(3-pyridyl)éthoxy]benzimidazole

### EXEMPLE 64 : 2-Phénoxyméthyl-5-[4-(diméthylamino)phénoxy]benzimidazole

### EXEMPLE 65 : 2-Phénoxyméthyl-5-[(3-pyridyl)thio]benzimidazole

### EXEMPLE 66 : Etude pharmacologique des dérivés de l'invention

Les composés ont été étudiés sur la lignée cellulaire humaine THP1 de type monocyte/macrophage. La production d'IL 1β par ces cellules a été obtenue après stimulation avec du lipopolysaccharide bactérien (*M. Turner et coll., Biochem. Biophys. Res. Comm., 1988, 256(2), 830-839*) et dosée par la méthode EIA (kit Cayman) selon les instructions fournies par le fabricant. Sur le test du choc endotoxique provoqué chez la souris par injection intraveineuse de lipopolysaccharide, les composés de l'invention ont diminué d'environ 50 % les taux circulants de TNFα pour une dose orale de 100 mg/kg.

### EXEMPLE 67 : Composition pharmaceutique

| Formule de préparation pour 1000 comprimés dosés à 10 mg | |
|---|---|
| Composé de l'exemple 2 | 10 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composé de formule (I) 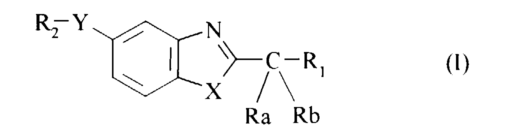 dans laquelle :
R₁ représente un atome d'halogène, un groupement hydroxy, alkoxy (C₁-C₆) linéaire ou ramifié (éventuellement substitué par un groupement aryle), trihalogénométhyle, amino (éventuellement substitué par un ou plusieurs groupements alkyle (C₁-C₆) linéaire ou ramifié, ou aryle éventuellement substitué), mercapto, alkylthio (C₁-C₆) linéaire ou ramifié, trialkylammonium (C₁-C₆) linéaire ou ramifié, aryloxy, arylthio, arylsulfonyle, arylsulfonyloxy, cycloalkyl (C₃-C₇) oxy, cycloalkyl (C₃-C₇) thio, bicycloalkyle (C₆-C₈) oxy substitué éventuellement par un groupement aryle, bicycloalkyl (C₆-C₈) thio éventuellement substitué par un groupement aryle,
Rₐ, R_{b}, identiques ou différents, représentent un atome d'hydrogène ou un groupement alkyle (C₁-C₆) linéaire ou ramifié,
X représente un atome d'oxygène ou de soufre ou un groupement NR (dans lequel R représente un atome d'hydrogène ou un groupement alkyle (C₁-C₆) linéaire ou ramifié),
Y représente un groupement -(CH₂)ₘ-Z-(CH₂)ₙ- dans lequel
m représente 0, 1 ou 2,
n représente 0, 1 ou 2,
Z représente un atome d'oxygène ou de soufre ou un groupement amino (éventuellement substitué par un groupement alkyle (C₁-C₆) linéaire ou ramifié), un groupement -SO₂-, -CHOH- ou -CH(CH₂OH)-,
R₂ représente un groupement aryle éventuellement substitué,
étant entendu que par aryle, on entend phényle, naphtyle, pyridyle, quinolyle, imidazolyle ou pyridyl-N-oxide,
et que par éventuellement substitué, on entend un ou plusieurs atomes d'halogène ou groupements alkyle (C1-C6) linéaire ou ramifié, trihalogénoalkyle (C1-C6) linéaire ou ramifié, alkoxy (C1-C6) linéaire ou ramifié, hydroxy, nitro, cyano, amino (éventuellement substitué par un ou plusieurs groupements alkyle (C1-C6)), phényle ou bicycloalkyle substitué par méthoxy
ses isomères ainsi que ses sels d'addition à un acide ou à une base pharmaceutiquement acceptable, à la condition que le composé de formule (I) soit différent du 2-méthoxyméthyl-5-(phénylthio)benzimidazole.

2. Composés de formule (I) selon la revendication 1 tels que X représente un groupement NR.

3. Composés de formule (I) selon la revendication 1 tels que Y représente un atome d'oxygène ou de soufre.

4. Composés de formule (I) selon la revendication 1 tels que R₁ représente un groupement hydroxy.

5. Composés de formule (I) selon la revendication 1 tels que R₁ représente un groupement mercapto.

6. Composés de formule (I) selon la revendication 1 tels que R₁ représente un groupement arylthio ou aryloxy éventuellement substitué.

7. Composés de formule (I) selon la revendication 6 tel que R₁ représente un groupement phénoxy ou phénylthio éventuellement substitué.

8. Composés de formule (I) selon la revendication 1 tels que R₂ représente un groupement phényle éventuellement substitué.

9. Composés de formule (I) selon la revendication 1 tels que R₂ représente un groupement pyridyle éventuellement substitué.

10. Composé de formule (I) selon la revendication 1 qui est le 2-hydroxyméthyl-5-(4-pyridyloxy)benzimidazole.

11. Composé de formule (I) selon la revendication 1 qui est le 2-phénoxyméthyl 5-(4-pyridylthio)benzimidazole.

12. Composé de formule (I) selon la revendication 1 qui est le 2-phénoxyméthyl-5-(N-méthyl-4-pyridylamino) benzimidazole.

13. Composé de formule (I) selon la revendication 1 qui est le 2-hydroxyméthyl-5-(4-pyridylméthoxy) benzimidazole.

14. Procédé de préparation des composés de formule (I) caractérisé lorsque les composés de formule (I) que l'on souhaite obtenir sont tels que X = NR, en ce que l'on utilise comme produit de départ un composé de formule (II) :
R₂-Y-H (II)
dans laquelle R₂ et Y sont tels que définis dans la formule( I), que l'on fait réagir sur la 2-nitro-5-chloroaniline,
pour conduire au composé de formule (IlI) : 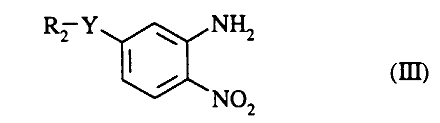 dans laquelle R₂ et Y sont tels que définis dans la formule (I), qui subit une réduction catalytique,
pour conduire au composé de formule (IV): 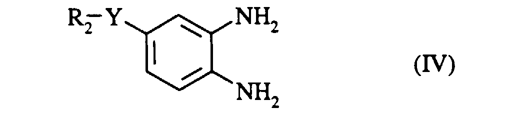 dans laquelle R₂ et Y sont tels que définis dans la formule (I),
que l'on fait réagir, en milieu acide, avec un composé de formule (V) : 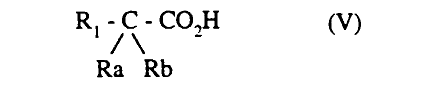 dans laquelle Rₐ, R_{b} et R₁ ont la même signification que dans la formule (I),
pour conduire au composé de formule (I/a), cas particulier des composés de formule (I) : 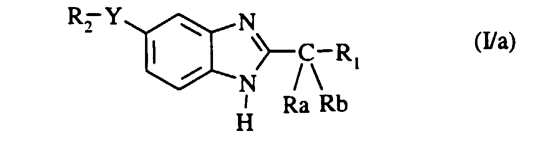 dans laquelle R₁, Rₐ, R_{b}, R₂ et Y sont tels que définis dans la formule (I),
qui subit, éventuellement lorsque R₁ représente un groupement hydroxy, l'action du chlorure de thionyle,
pour conduire au composé de formule (I/b), cas particulier des composés de formule (I) : 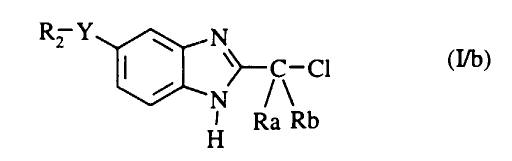 dans laquelle R₂, Y, Rₐ et R_{b} ont la même signification que dans la formule (I),
qui peut alors subir les réactions classiques que l'on peut mettre en oeuvre sur dérivés chlorés, pour obtenir les substitutions correspondantes,
composé de formule (I/a) ou (I/b) :
- dont la fonction NH peut être éventuellement substituée par un groupement alkyle (C₁-C₆) linéaire ou ramifié,
- qui peut être, le cas échéant, purifié selon une technique classique de purification
- dont on sépare, le cas échéant, les isomères selon une technique classique de séparation,
- que l'on transforme, si on le souhaite, en ses sels d'addition à une base pharmaceutiquement acceptable.

15. Procédé de préparation caractérisé lorsque les composés de formule (I) que l'on souhaite obtenir sont tels que X = X' = O ou S, en ce que l'on utilise comme produit de départ un composé de formule (VI) : 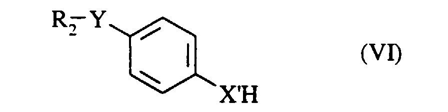 dans lequel R₂ et Y sont tels que définis dans la formule (I) et X' représente un atome d'oxygène ou de soufre, sur lequel on fait réagir de l'acide nitrique,
pour conduire au composé de formule (VII) : 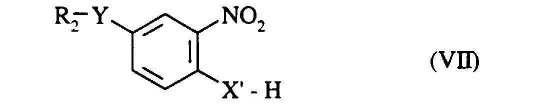 dans laquelle R₂, Y et X' sont tels que définis précédemment, qui subit une hydrogénation catalytique,
pour conduire au composé de formule (VIII) : 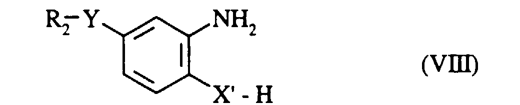 que l'on fait réagir, en milieu acide, avec un composé de formule (V) : 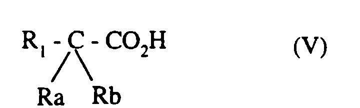 dans laquelle Rₐ, R_{b} et R₁ ont la même signification que dans la formule (I),
pour conduire au composé de formule (I/c), cas particulier des composés de formule (I) : 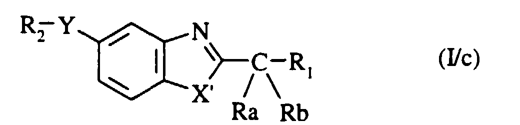 dans laquelle R₂, Y, X', Rₐ, R_{b} et R₁ sont tels que définis précédemment,
qui subit, éventuellement lorsque R₁ représente un groupement hydroxy, l'action du chlorure de thionyle,
pour conduire au composé de formule (I/d), cas particulier des composés de formule (I) : 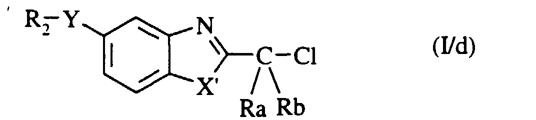 dans laquelle R₂, X', Y, Rₐ et R_{b} ont la même signification que précédemment,
qui peut alors subir les réactions classiques que l'on peut mettre en oeuvre sur des dérivés chlorés, pour obtenir les substitutions correspondantes,
composé de formule (I/c) ou (I/d) :
- qui peut être, le cas échéant, purifié selon une technique classique de purification
- dont on sépare, le cas échéant, les isomères selon une technique classique de séparation,
- que l'on transforme, si on le souhaite, en ses sels d'addition à une base pharmaceutiquement acceptable.

16. Compositions pharmaceutiques contenant comme principe actif au moins un composé selon l'une quelconque des revendications 1 à 13, seul ou en combinaison avec un ou plusieurs excipients ou véhicules inertes, non toxiques, pharmaceutiquement acceptables.

17. Compositions pharmaceutiques selon la revendication 16 contenant au moins un principe actif selon l'une quelconque des revendications 1 à 13 utiles comme inhibiteurs d'interleukine 1β.

## Patentansprüche

1. Verbindung der Formel (I): 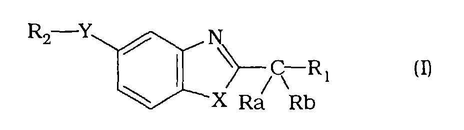 in der:
R₁ ein Halogenatom, eine Hydroxygruppe, geradkettige oder verzweigte (gegebenenfalls durch eine Arylgruppe substituierte) (C₁C₆)-Alkoxygruppe, Trihalogenmethylgruppe, (gegebenenfalls durch eine oder mehrere geradkettige oder verzweigte (C₁-C₆)-Alkylgruppen oder eine gegebenenfalls substituierte Arylgruppe substituierte) Aminogruppe, Mercaptogruppe, geradkettige oder verzweigte (C₁-C₆)-Alkylthiogruppe, geradkettige oder verzweigte (C₁-C₆)-Trialkylammoniumgruppe, Aryloxygruppe, Arylthiogruppe, Arylsulfonylgruppe, Arylsulfonyloxygruppe, (C₃-C₇)-Cycloalkyl-oxygruppe, (C₃-C₇)-Cycloalkyl-thiogruppe, gegebenenfalls durch eine Arylgruppe substituierte (C₆-C₈)-Bicycloalkyl-oxygruppe oder gegebenenfalls durch eine Arylgruppe substituierte (C₆-C₈)-Bicycloalkyl-thiogruppe,
Rₐ, R_{b}, die gleichartig oder verschieden sind, jeweils ein Wasserstoffatom oder eine geradkettige oder verzweigte (C₁-C₆)-Alkylgruppe,
X ein Sauerstoffatom oder ein Schwefelatom oder eine Gruppe NR (worin R ein Wasserstoffatom oder eine geradkettige oder verzweigte (C₁-C₆)-Alkylgruppe darstellt),
Y eine Gruppe der Formel -(CH₂)ₘ-Z-(CH₂)ₙ-, in der:
m 0, 1 oder 2,
n 0, 1 oder 2 und
Z ein Sauerstoffatom oder ein Schwefelatom oder eine (gegebenenfalls durch eine geradkettige oder verzweigte (C₁-C₆)-Alkylgruppe substituierte) Aminogruppe oder eine Gruppe -SO₂-, -CHOH- oder -CH(CH₂OH)-darstellen, und
R₂ eine gegebenenfalls substituierte Arylgruppe bedeuten,
mit der Maßgabe, daß unter einer Arylgruppe eine Phenyl-, Naphthyl-, Pyridyl-, Chinolyl-, Imidazolyl- oder Pyridyl-N-oxidgruppe zu verstehen ist
und daß unter gegebenenfalls substituiert ein oder mehrere Halogenatome oder geradkettige oder verzweigte (C₁-C₆)-Alkylgruppen, geradkettige oder verzweigte (C₁-C₆)-Trihalogenalkylgruppen, geradkettige oder verzweigte (C₁-C₆)-Alkoxygruppen, Hydroxygruppen, Nitrogruppen, Cyanogruppen, (gegebenenfalls durch eine oder mehrere (C₁-C₆)-Alkylgruppen substituierte) Aminogruppen, und durch Methoxygruppen substituierte Phenyl- oder Bicycloalkylgruppen zu verstehen sind, deren Isomere sowie deren Additionssalze mit einer pharmazeutisch annehmbaren Säure oder Base,
mit der Maßgabe, daß die Verbindung der Formel (I) von 2-Methoxymethyl-5-(phenylthio)-benzimidazol verschieden ist.

2. Verbindungen der Formel (I) nach Anspruch 1, worin X eine Gruppe NR darstellt.

3. Verbindungen der Formel (I) nach Anspruch 1, worin Y ein Sauerstoff- oder Schwefelatom darstellt.

4. Verbindungen der Formel (I) nach Anspruch 1, worin R₁ eine Hydroxygruppe darstellt.

5. Verbindungen der Formel (I) nach Anspruch 1, worin R₁ eine Mercaptogruppe darstellt.

6. Verbindungen der Formel (I) nach Anspruch 1, worin R₁ eine gegebenenfalls substituierte Arylthiogruppe oder Aryloxygruppe darstellt.

7. Verbindungen der Formel (I) nach Anspruch 6, worin R₁ eine gegebenenfalls substituierte Phenoxygruppe oder Phenylthiogruppe darstellt.

8. Verbindungen der Formel (I) nach Anspruch 1, worin R₂ eine gegebenenfalls substituierte Phenylgruppe darstellt.

9. Verbindungen der Formel (I) nach Anspruch 1, worin R₂ eine gegebenenfalls substituierte Pyridylgruppe darstellt.

10. Verbindung der Formel (I) nach Anspruch 1, nämlich 2-Hydroxymethyl-5-(4-pyridyloxy)-benzimidazol.

11. Verbindung der Formel (I) nach Anspruch 1, nämlich 2-Phenoxymethyl-5-(4-pyridylthio)-benzimidazol.

12. Verbindung der Formel (I) nach Anspruch 1, nämlich 2-Phenoxymethyl-5-(N-methyl-4-pyridylamino)-benzimidazol.

13. Verbindung der Formel (I) nach Anspruch 1, nämlich 2-Hydroxymethyl-5-(4-pyridylmethoxy)-benzimidazol.

14. Verfahren zur Herstellung der Verbindungen der Formel (I), dadurch gekennzeichnet, daß man, wenn die Verbindungen der Formel (I), die man herzustellen wünscht, jene sind, worin X = NR bedeutet, als Ausgangsprodukt eine Verbindung der Formel (II) einsetzt:
R₂ - Y - H (II)
in der R₂ und Y die bezüglich der Formel (I) angegebenen Bedeutungen besitzen,
welche man mit 2-Nitro-5-chloranilin umsetzt,
zur Bildung der Verbindung der Formel (III): 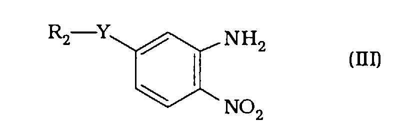 in der R₂ und Y die bezüglich der Formel (I) angegebenen Bedeutungen besitzen,
welche man einer katalytischen Reduktion unterzieht,
zur Bildung der Verbindung der Formel (IV): 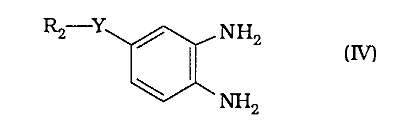 in der R₂ und Y die bezüglich der Formel (I) angegebenen Bedeutungen besitzen,
welche man in saurem Medium mit einer Verbindung der Formel (V) umsetzt: 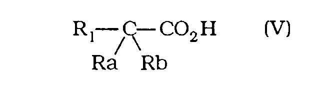 in der Rₐ, R_{b} und R₁ die bezüglich der Formel (I) angegebenen Bedeutungen besitzen,
zur Bildung der Verbindung der Formel (I/a), einem Sonderfall der Verbindungen der Formel (I): 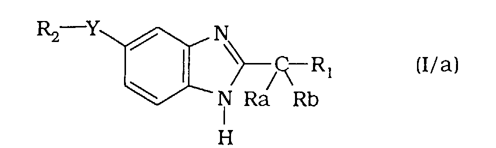 in der R₁, Rₐ, R_{b}, R₂ und Y die bezüglich der Formel (I) angegebenen Bedeutungen besitzen,
welche man, wenn R₁ eine Hydroxygruppe darstellt, der Einwirkung von Thionylchlorid unterzieht,
zur Bildung der Verbindung der Formel (I/b), einem Sonderfall der Verbindungen der Formel (I): 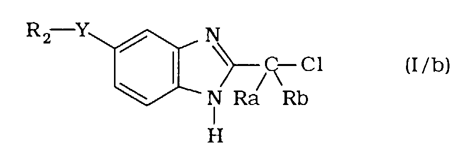 in der R₂, Y, Rₐ und R_{b} die bezüglich der Formel (I) angegebenen Bedeutungen besitzen,
welche man dann den klassischen Reaktionen unterwerfen kann, bei denen man chlorierte Derivate einsetzen kann zur Bildung der entsprechenden substituierten Verbindungen,
wobei die Verbindung der Formel (I/a) oder (I/b):
- an der NH-Funktion gegebenenfalls durch eine geradkettige oder verzweigte (C₁-C₆)-Alkylgruppe substituiert werden kann,
- gegebenenfalls mit Hilfe einer klassischen Reinigungsmethode gereinigt werden kann,
- man gegebenenfalls die Isomeren mit Hilfe einer klassischen Trennmethode trennen kann und
- die man gewünschtenfalls mit einer pharmazeutisch annehmbaren Base in ihre Säureadditionssalze umwandeln kann.

15. Verfahren zur Herstellung der Verbindungen der Formel (I), in der X = X' = O oder S bedeutet, dadurch gekennzeichnet, daß man als Ausgangsprodukt eine Verbindung der Formel (VI) einsetzt: 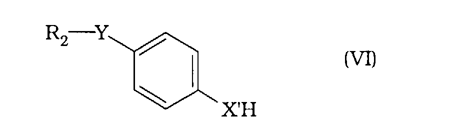 in der R₂ und Y die bezüglich der Formel (I) angegebenen Bedeutungen besitzen und X' ein Sauerstoff- oder Schwefelatom bedeutet, welche man mit Salpetersäure umsetzt,
zur Bildung der Verbindung der Formel (VII): 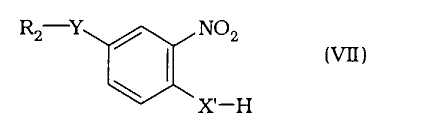 in der R₂, Y und X' die oben angegebenen Bedeutungen besitzen, welche man einer katalytischen Hydrierung unterzieht,
zur Bildung der Verbindung der Formel (VIII): 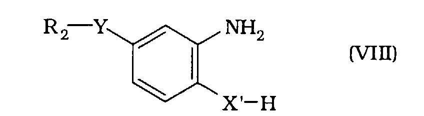 welche man in saurem Medium mit einer Verbindung der Formel (V) umsetzt: 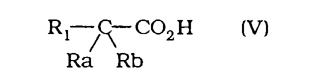 in der Rₐ, R_{b} und R₁ die bezüglich der Formel (I) angegebenen Bedeutungen besitzen,
zur Bildung der Verbindung der Formel (I/c), einem Sonderfall der Verbindungen der Formel (I): 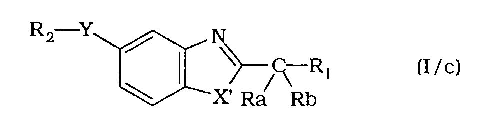 in der R₂, Y, X', Rₐ, R_{b} und R₁ die oben angegebenen Bedeutungen besitzen,
welche man, wenn R eine Hydroxygruppe darstellt, gegebenenfalls der Einwirkung von Thionylchlorid unterwirft,
zur Bildung der Verbindung der Formel (I/d), einem Sonderfall der Verbindungen der Formel (I): 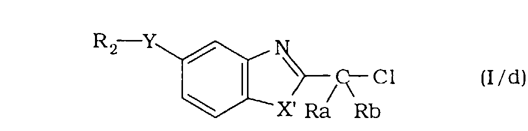 in der R₂, X', Y, Rₐ und R_{b} die oben angegebenen Bedeutungen besitzen,
welche dann den klassischen Reaktionen, bei denen man chlorierte Derivate einsetzen kann, unterworfen werden kann, zur Bildung der entsprechenden substituierten Verbindungen,
welche Verbindung der Formel (I/c) oder (I/d):
- gegebenenfalls mit Hilfe einer klassischen Reinigungsmethode gereinigt werden kann,
- gegebenenfalls mit Hilfe einer klassischen Trennmethode in ihre Isomeren getrennt werden kann und
- gewünschtenfalls mit einer pharmazeutisch annehmbaren Base in ihre Additionssalze umgewandelt werden kann.

16. Pharmazeutische Zubereitungen enthaltend als Wirkstoff mindestens eine Verbindung nach einem der Ansprüche 1 bis 13 allein oder in Kombination mit einem oder mehreren inerten, nichttoxischen, pharmazeutisch annehmbaren Trägermaterialien oder Bindemitteln.

17. Pharmazeutische Zubereitungen nach Anspruch 16, enthaltend mindestens einen Wirkstoff nach einem der Ansprüche 1 bis 13 als Inhibitoren von Interleukin 1β.

## Claims

1. Compound of formula (I): 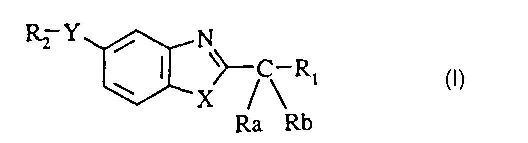 in which:
R₁ represents a halogen atom, a hydroxy group, a linear or branched (C₁-C₆)-alkoxy group (optionally substituted by an aryl group), a trihalomethyl group, an amino group (optionally substituted by one or more linear or branched (C₁-C₆)alkyl or optionally substituted aryl groups), a mercapto group, a linear or branched (C₁-C₆)alkylthio group, a linear or branched tri-(C₁-C₆)alkylammonium group, an aryloxy group, an arylthio group, an arylsulphonyl group, an arylsulphonyloxy group, a (C₃-C₇)cycloalkyloxy group, a (C₃-C₇)cycloalkylthio group, a di-(C₆-C₈)cycloalkyloxy group optionally substituted by an aryl group, or a di-(C₆-C₈)cycloalkylthio group optionally substituted by an aryl group,
Rₐ, R_{b}, which may be identical or different, represent a hydrogen atom or a linear or branched (C₁-C₆)alkyl group,
X represents an oxygen or sulphur atom or an NR group (in which R represents a hydrogen atom or a linear or branched (C₁-C₆)alkyl group),
Y represents a -(CH₂)ₘ-Z-(CH₂)ₙ- group in which:
m represents 0, 1 or 2,
n represents 0, 1 or 2,
Z represents an oxygen or sulphur atom or an amino group (optionally substituted by a linear or branched (C₁-C₆)alkyl group), a -SO₂-, -CHOH- or -CH(CH₂OH)- group,
R₂ represents an optionally substituted aryl group,
wherein aryl is to be understood as meaning phenyl, naphthyl, pyridyl, quinolyl, imidazolyl or pyridyl N-oxide,
and wherein optionally substituted is to be understood as meaning one or more halogen atoms or linear or branched (C₁-C₆)alkyl groups, linear or branched (C₁-C₆)-trihaloalkyl groups, linear or branched (C₁-C₆)alkoxy groups, hydroxy groups, nitro groups, cyano groups, amino groups (optionally substituted by one or more (C₁-C₆)-alkyl groups), or phenyl or bicycloalkyl groups substituted by methoxy,
its isomers and addition salts thereof with a pharmaceutically acceptable acid or base, with the proviso that the compound of formula (I) is other than 2-methoxy-methyl-5-(phenylthio)benzimidazole.

2. Compounds of formula (I) according to claim 1 wherein X represents an NR group.

3. Compounds of formula (I) according to claim 1 wherein Y represents an oxygen or sulphur atom.

4. Compounds of formula (I) according to claim 1 wherein R₁ represents a hydroxy group.

5. Compounds of formula (I) according to claim 1 wherein R₁ represents a mercapto group.

6. Compounds of formula (I) according to claim 1 wherein R₁ represents an optionally substituted arylthio or aryloxy group.

7. Compounds of formula (I) according to claim 6 wherein R₁ represents an optionally substituted phenoxy or phenylthio group.

8. Compounds of formula (I) according to claim 1 wherein R₂ represents an optionally substituted phenyl group.

9. Compounds of formula (I) according to claim 1 wherein R₂ represents an optionally substituted pyridyl group.

10. Compound of formula (I) according to claim 1 which is 2-hydroxymethyl-5-(4-pyridyloxy)benzimidazole.

11. Compound of formula (I) according to claim 1 which is 2-phenoxymethyl-5-(4-pyridylthio)benzimidazole.

12. Compound of formula (I) according to claim 1 which is 2-phenoxymethyl-5-(N-methyl-4-pyridylamino)benzimidazole.

13. Compound of formula (I) according to claim 1 which is 2-hydroxymethyl-5-(4-pyridylmethoxy)benzimidazole.

14. Process for the preparation of compounds of formula (I), characterised, when the compounds of formula (I) to be obtained are those wherein X = NR, in that there is used as starting material a compound of formula (II):
R₂ - Y - H (II)
in which R₂ and Y are as defined in formula (I), which is reacted with 2-nitro-5-chloroaniline
to yield a compound of formula (III): 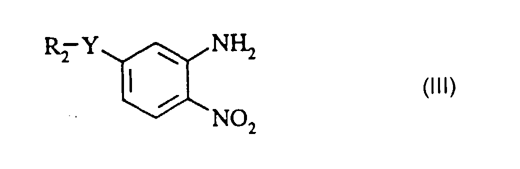 in which R₂ and Y are as defined in formula (I), which is subjected to catalytic reduction
to yield a compound of formula (IV): 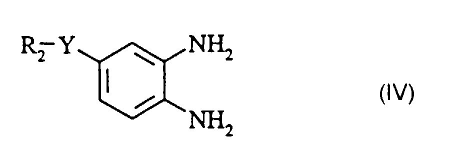 in which R₂ and Y are as defined in formula (I),
which is reacted, in an acid medium, with a compound of formula (V): 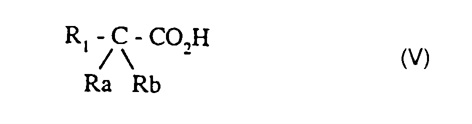 in which Rₐ, R_{b} and R₁ are as defined in formula (I)
to yield a compound of formula (I/a), a particular case of the compounds of formula (I): 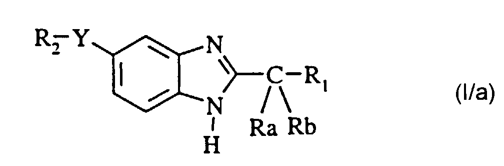 in which R₁, Rₐ, R_{b}, R₂ and Y are as defined in formula (I),
which is optionally subjected, when R₁ represents a hydroxy group, to the action of thionyl chloride
to yield a compound of formula (I/b), a particular case of the compounds of formula (I): 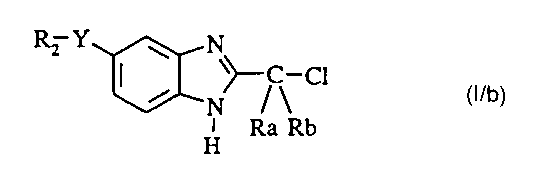 in which R₂, Y, Rₐ and R_{b} are as defined in formula (I),
which may then be subjected to the conventional reactions which may be carried out on chlorinated compounds, to obtain the corresponding substitutions,
which compound of formula (I/a) or (I/b):
- the NH function of which may optionally be substituted with a linear or branched (C₁-C₆)alkyl group,
- may, where appropriate, be purified by a conventional purification technique,
- is separated, where appropriate, into its isomers by a conventional separation technique,
- is converted, if desired, into its addition salts with a pharmaceutically acceptable base.

15. Preparation process characterised, when the compounds of formula (I) to be obtained are those wherein X = X' = O or S, in that there is used as starting material a compound of formula (VI): 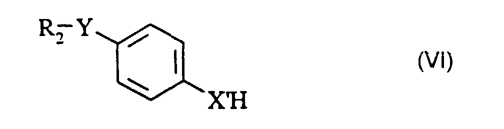 in which R₂ and Y are as defined in formula (I) and X' represents an oxygen or sulphur atom, which is reacted with nitric acid
to yield a compound of formula (VII): 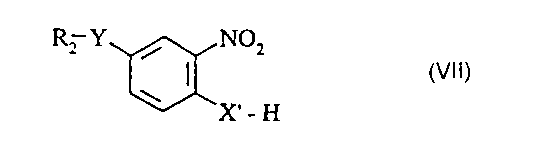 in which R₂, Y and X' are as defined above, which is subjected to catalytic hydrogenation
to yield a compound of formula (VIII): 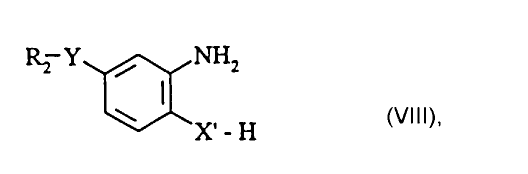 which is reacted, in an acid medium, with a compound of formula (V): 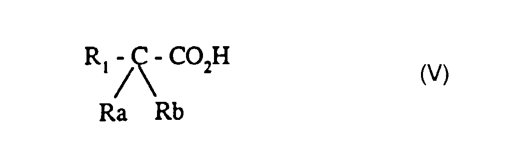 in which Rₐ, R_{b} and R₁ are as defined in formula (I)
to yield a compound of formula (I/c), a particular case of the compounds of formula (I): 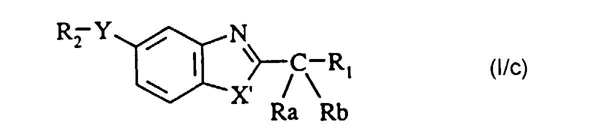 in which R₂, Y, X', Rₐ, R_{b} and R₁ are as defined above,
which is optionally subjected, when R₁ represents a hydroxy group, to the action of thionyl chloride
to yield a compound of formula (I/d), a particular case of the compounds of formula (I): 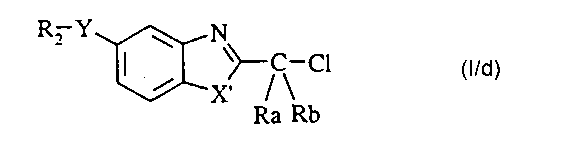 in which R₂, X', Y, Rₐ and R_{b} are as defined above,
which may then be subjected to the conventional reactions which may be carried out on chlorinated compounds, to obtain the corresponding substitutions, which compound of formula (I/c) or (I/d):
- may, where appropriate, be purified by a conventional purification technique,
- is separated, where appropriate, into its isomers by a conventional separation technique,
- is converted, if desired, into its addition salts with a pharmaceutically acceptable base.

16. Pharmaceutical compositions comprising as active ingredient at least one compound according to any one of claims 1 to 13, alone or in combination with one or more inert, non-toxic, pharmaceutically acceptable excipients or carriers.

17. Pharmaceutical compositions according to claim 16 comprising at least one active ingredient according to any one of claims 1 to 13 for use as inhibitors of interleukin-1β.
